# EUROPEAN PATENT SPECIFICATION

(11) **EP 2 526 936 B1**
(45) Date of publication and mention of the grant of the patent: **15.04.2015**
(21) Application number: 11167114.5
(22) Date of filing: 23.05.2011
(51) Int. Cl.: A61K 9/14, A61K 9/16, A61K 31/23

(54) **Particle size distribution of cetyl myristate and/or cetyl palmitate**
Partikelgrößenverteilung für Cetyl myristat und / oder Cetyl Palmitat
Repartition granulometrique de cetyl myristate et/ou cetyl palmitate

(43) Date of publication of application: 28.11.2012
(73) Proprietor: Deva Holding Anonim Sirketi, 34303 Istanbul (TR)
(72) Inventor: Haas, Philipp Daniel, 34303, ISTANBUL (TR); Firat, Omer Faruk, 34303, ISTANBUL (TR); Kandemir, Levent, 34303, ISTANBUL (TR); Koc, Fikret, 34303, ISTANBUL (TR); Sivasligil, Ramazan, 34303, ISTANBUL (TR)

(56) References cited:
- WO-A1-2004/062643
- ANONYMOUS: "Cetyl Myristoleate Powder Specification Sheet", INTERNET CITATION, 31 July 2007 (2007-07-31), pages 1-2, XP002632858, Retrieved from the Internet: URL:http://www.essentiallypure.com/cetyl_m yristoleate_spec.htm [retrieved on 2011-04-14]

## Description

### Technical Field

This invention is related to defining critical particle size distribution and flowability characteristics of granules and/or powder mixtures of cetyl myristate or cetyl palmitate or combination of cetyl myristate and cetyl palmitate and optimizing process parameters for manufacturing.

### Background Art

Cetyl palmitate is derived from the fatty acid, palmitic acid which occurs as the glycerol ester in many oils and fats such as palm oil or Chinese vegetable tallow. A synthetic method of preparation is to reacting palmitoyl chloride and cetyl alcohol in the presence of magnesium. See the Merck Index, 12th edition at page 336. Reference is also made to US 3169099 A (SOCONY MOBIL OIL CO INC) 02.09.1965 patent which discloses a biosynthetic method of producing cetyl palmitate.

Cetyl myristate is derived from the saturated fatty acid, myristic acid. This acid is found in nutmeg butter, in fats of Myristicaceae, in palm seed fats, milk fats and also sperm whale oil.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt. % of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate. That mixture comprises by weight 95% cetyl myristate and 5% cetyl palmitate by weight. In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/ or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate. The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt. %) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention embraces critical particle size distribution, flowability characteristics of granules or powder mixtures comprising cetyl myristate or cetyl palmitate or combination of cetyl myristate and cetyl palmitate and related process development.

### Technical Problem

In the pharmaceutical industry, particle size distribution is one of the critical parameter in the manufacturing of many pharmaceutical products and directly influences physical properties of the final blend such as powder flowability, compressibility, homogeneity etc.

At manufacturing stages, poor flowability characteristics of formulated blend may lead to powder segregation and frequently results in content uniformity problems in finished products. Mismatch of drug and excipient particle size is a leading cause of segregation and poor content uniformity, and can be surpassed by properly defined particle size distribution of the blend.

Controlling the granulation particle size is important for maintaining an appropriate granulation flow, weight control during tabletting and minimize the likelihood of segregation.

A non uniform particle size distribution influences compressibility as well. When taking effects of hardness and weight variation into consideration, optimization of size distribution of granules is highly required on a larger scale.

Besides it is known that the manufacturability may be improved by optimizing particle size distribution and powder flowability of the formulated blend without changing the formulation.

Moreover, some parameters such as compressibility index, are most commonly used parameters for interparticulate interactions in the characterization of powder flowability.

Cetyl palmitate and cetyl myristate are both difficult-to-process drug substances due to their stickiness. Such drug substance like cetyl palmitate and cetyl myristate may impair the manufacturing in various steps since they might liquefy and becoming sticky even at ambient temperature with/without any mechanical impact.

Powdered or granular forms of cetyl palmitate and cetyl myristate tend to be oily to touch and not freely flowing which present problems in development and production of all solid dosage forms. Therefore, formulation and manufacture of these waxy compositions are extremely difficult by using conventional solid dosage approaches on both small and commercial scales.

Tablet manufacturing requires continuous and uniform flow of powder from feeder to the die cavities. Tablet pressing of cetyl palmitate and cetyl myristate granules and/or powder blend is very challenging, die and punch sticking becomes noticeable to an unacceptable degree. Especially in the case of waxy compositions, at high speed compression tablet weight variation may occur due to non homogeneous powder feeding to dies. Besides, at low speed compression, longer dwell time may cause the substance to melt as the heat resulting from compression.

Furthermore, size distribution of particles is a major concern for a successful capsule filing operation. Fine particles with high bulk density require a larger capsule which is particularly not suitable in high load drug formulation. In the case of coarse granules, filing within specification limits becomes impossible.

### Solution to Problem

It is desired to optimize particle size distribution and manufacturing process of oral solid dosage forms of cetyl myristate or cetyl palmitate or combination of cetyl myristate and cetyl palmitate and the methods of their preparation on a commercial scale.

In the manufacturing of cetyl myristate or cetyl palmitate or combination of cetyl myristate and cetyl palmitate oral dosage forms, known techniques remain insufficient by cause of their waxy characteristics. Subsequently, new process with critical parameters and process optimization for manufacturing are needful to develop.

The inventors of the present invention investigated effect of particle size distribution of cetyl myristate or cetyl palmitate or combination of cetyl myristate and cetyl palmitate pharmaceutical composition and surprisingly found that manufacturability problems are solved at both small and commercial scale with a final blend having determined particle size distribution. Also, inventors optimized related process parameters which affect critical particle size distribution.

### Description of embodiments

Aspects of the present invention relate to particle size distribution of pharmaceutical or dietary supplement formulations comprising cetyl myristate or cetyl palmitate or combination of cetyl myristate and cetyl palmitate.

Due to their oily features, sieving cetyl myristate or cetyl palmitate or combination of cetyl myristate and cetyl palmitate or other cohesive powders or granules is extremely difficult because of their affinity to clog sieve openings since mechanical force application for size reduction may destroy integrity of granules and result in melting of cetyl palmitate or cetyl myristate or combination thereof.

Homogenous particle size distribution of final blend can be achieved by milling or other methods for altering particle sizes as conventional methods. Conventional comminution techniques may be used for example grinding in air jet mill or impact mill, a ball mill, vibration mill, mortar mill and pin mill.

In the present invention, particle size distribution is determined according to US Pharmacopeia General Chapter <786>.

In another aspect of the present invention, physical properties of solid particles of cetyl myristate and cetyl palmitate are disclosed.

Compressibility index is indirect mesure of the flowability of the powder and it is determined according to US Pharmacopeia General Chapter <1174>.

For process optimization studies, a risk analysis was conducted on all process parameters of each unit operation. The high risk parameters were identified depending on their potential impact and probability of occurrence on drug product quality attributes. Parameters were selected as high risk factors to be studied using experimental design in order to optimize them.

A DOE (Design of Experiments) approach was used for process optimization, where the acceptable ranges around the target parameters for drying and filling were identified and studied.

A detailed analysis of in-process samples from each unit operation was conducted to ensure extensive characterization of the manufacturing process. In addition quality attributes of the finished drug product were measured and all responses were statistically analyzed.

Risk analysis was conducted by identifying the critical process parameters within each unit operation. The potential impact of the identified parameters on the critical quality attributes of the drug product and probability of occurrence were evaluated based on prior experience and knowledge.

Flow characteristics of granules and disintegration and dissolution of finished dosage form containing cetyl myristate or cetyl palmitate or combination of cetyl myristate and cetyl palmitate are particularly dependent on particle size distribution.

The drying had to be done at ambient temperature. Temperature, though identified as a high risk factor has not been included in the optimization plan since it has been optimized at 35°C. Drying time was identified as high risk factor for LOD and residual solvent. Therefore, it is necessary to optimize the drying time to ensure the quality of the product.

A randomized Fractional Factorial Design (2⁵⁻¹) with 3 center points (16+3=19 batches) was generated using Design Expert (Ver.8) software. Prior to running the DOE study, two trial batches were studied at the 2 kg batch size to ensure feasibility of manufacture. Run # 3 and Run#10 were selected for the trial batches as these represented extremes i.e. wettest and driest conditions respectively.

The results of the 19 batches have been summarized as below;

**Table 1**

| **Granules parameters** | | | | | | |
|---|---|---|---|---|---|---|
| **Run No** | **1** | **2** | **3** | **4** | **5** | **6** |
| | | | | | | |
| **LOD at 35°C (%)** | 0.13 | 0.30 | 0.05 | 0.31 | 0.37 | 0.12 |
| **Compressib ility Index (%)** | 19.15 | 14.58 | 19.77 | 13.83 | 19.15 | 15.61 |

| **Particle Size Distribution (% Granules retained on µm)** | | | | | | |
|---|---|---|---|---|---|---|
| **# 1700 µm (%)** | 16.5 | 12.2 | 8.58 | 9.14 | 12.44 | 12.36 |
| **# 425 µm (%)** | 45.94 | 40.66 | 5.58 | 49.6 | 34.38 | 34.88 |
| **# 250 µm (%)** | 22.16 | 29.8 | 57.9 | 27.02 | 28.52 | 28.94 |
| **# 180 µm (%)** | 5.56 | 5.38 | 9.24 | 6.64 | 5.6 | 12.06 |
| **# 150 µm (%)** | 5.68 | 6.88 | 9.66 | 5.54 | 7.8 | 7.2 |
| **Below # 150 µm (%)** | 3.64 | 4.72 | 7.2 | 2 | 10.8 | 3.32 |

**Table 2**

| **Granules parameters** | | | | | | |
|---|---|---|---|---|---|---|
| **Run No** | **7** | **8** | **9** | **10** | **11** | **12** |
| **LOD at 35°C (%)** | 0.84 | 0.12 | 0.23 | 0.05 | 0.07 | 0.67 |
| **Compressib ility Index (%)** | 12.77 | 11.83 | 11.83 | 11.11 | 12.5 | 12.5 |

| **Particle Size Distribution (% Granules retained on µm)** | | | | | | |
|---|---|---|---|---|---|---|
| **# 1700 µm (%)** | 9.16 | 12.74 | 5.7 | 10.56 | 6.12 | 8.1 |
| **# 425 µm (%)** | 48.78 | 42.48 | 36.68 | 50.12 | 43.66 | 44.5 |
| **# 250 µm (%)** | 31.32 | 27.62 | 30.16 | 25.46 | 34.02 | 31.06 |
| **# 180 µm (%)** | 4.52 | 6.44 | 10.4 | 4.48 | 7.12 | 8.18 |
| **# 150 µm (%)** | 4.58 | 6.14 | 12.12 | 5.34 | 5.92 | 5.22 |
| **Below # 150µm(%)** | 1.56 | 2.14 | 5 | 2.34 | 1.88 | 2.74 |

**Table 3**

| **Granules parameters** | | | | | | |
|---|---|---|---|---|---|---|
| **Run No** | **13** | **14** | **15** | **16** | **17** | **18** |
| **LOD at 35°C (%)** | 0.18 | 0.08 | 0.16 | 0.07 | 0.07 | 0.03 |
| **Compressib ility Index (%)** | 14.13 | 15.05 | 12.05 | 17.05 | 17.90 | 17.39 |

| **Particle Size Distribution (% Granules retained on µm)** | | | | | | |
|---|---|---|---|---|---|---|
| **# 1700 µm (%)** | 7.08 | 10.36 | 6.46 | 11 | 12.48 | 8.82 |
| **# 425 µm (%)** | 46.78 | 53.08 | 42.94 | 36.62 | 38.28 | 38.1 |
| **# 250 µm (%)** | 32.84 | 27.36 | 34.42 | 32.1 | 28.42 | 26.26 |
| **#180 µm (%)** | 5.88 | 3.88 | 6.64 | 8.74 | 10.62 | 19.18 |
| **#150 µm (%)** | 5.22 | 3.84 | 6.1 | 7.08 | 6.02 | 5.26 |
| **Below # 150µm(%)** | 1.88 | 1.12 | 2.68 | 4.22 | 4 | 2.32 |

**Table 4**

| **Granules parameters** | |
|---|---|
| **Run No** | **19** |
| **LOD at 35°C (%)** | 0.13 |
| **Compressibility Index (%)** | 13.33 |

| Particle Size Distribution (% Granules retained on µm) | |
|---|---|
| **# 1700 µm(%)** | 9.4 |
| **# 425 µm(%)** | 49.54 |
| **# 250 µm(%)** | 29.68 |
| **# 180 µm(%)** | 4.78 |
| **# 150 µm(%)** | 5.42 |
| **Below # 150µm(%)** | 2.88 |

These values are useful for generating specifications or limits for semi-finished or finished dosage forms.

In a first aspect, pharmaceutical or dietary supplement formulations comprising cetyl myristate or cetyl palmitate or combination of cetyl myristate and cetyl palmitate particles wherein particle size distribution is in the range of 150 µm and 1600 µm. The pharmaceutical or dietary supplement formulations of cetyl myristate or cetyl palmitate or combination of cetyl myristate and cetyl palmitate characterized in that said formulations are prepared by using of: a) particles of cetyl myristate or cetyl palmitate or combination of cetyl myristate and cetyl palmitate or, b) powder mixtures of cetyl myristate or cetyl palmitate or combination of cetyl myristate and cetyl palmitate with an excipient or mixtures of excipients or, c) granules of cetyl myristate or cetyl palmitate or combination of cetyl myristate and cetyl palmitate having particle size distribution in the range of 150 µm ≤PSD≤ 1600 µm.

In another aspect, pharmaceutical or dietary supplement formulations comprising cetyl myristate or cetyl palmitate or combination of cetyl myristate and cetyl palmitate have particle size distribution wherein about 95% of particles are larger than 150 µm.

In another aspect, pharmaceutical or dietary supplement formulations comprising cetyl myristate or cetyl palmitate or combination of cetyl myristate and cetyl palmitate have particle size distribution wherein about %25 of particles are between 150 µm and 250 µm.

In another aspect, pharmaceutical or dietary supplement formulations comprising cetyl myristate or cetyl palmitate or combination of cetyl myristate and cetyl palmitate have particle size distribution wherein about 60% of particles are between 250 µm and 425µm.

In another aspect, pharmaceutical or dietary supplement formulations comprising cetyl myristate or cetyl palmitate or combination of cetyl myristate and cetyl palmitate have particle size distribution wherein about 60% of particles are larger than 425 µm.

Three characteristic particle classes are defined as follow; ultrafine (corresponds to feed powder having diameter less or equal to 150µm); fine (corresponds to feed powder having diameter between 150 µm and 250 µm); coarse (corresponds to feed powder having diameter greater than 425 µm.

According to this invention, a) particles of cetyl myristate or cetyl palmitate or combination of cetyl myristate and cetyl palmitate or, b) powder mixtures of cetyl myristate or cetyl palmitate or combination of cetyl myristate and cetyl palmitate with an excipient or mixtures of excipients or, c) granules of cetyl myristate or cetyl palmitate or combination of cetyl myristate and cetyl palmitate are characterized in that a-) ratio of quantity of ultrafine particles to quantity of coarse particles is 2:1 to 1:6, b-) ratio of quantity of coarse particles to quantity of fine particles is 5:1 to 5:12, c-) ratio of quantity of coarse particles to quantity of cumulative total particles is 1:19 to 3:2, d-) ratio of quantity of particles with particles larger than 250 µm to quantity of particles smaller than 250 µm is 1:1 to 19:1.

Ratio of quantity of ultrafine particles (particles≤150 µm) to quantity of coarse particles (particles>425 µm) is 2:1 to 1:6.

Ratio of quantity of coarse particles (particles >425 µm) to quantity of fine particles (particies 150µm -250µm) is 5:1 to 5:12.

Ratio of quantity of coarse particles (particles >425 µm) to quantity of cumulative total particles (particles<425 µm) is 1:19 to 3:2.

Ratio of quantity of particles with particles larger than 250 µm to quantity of particles smaller than 250 µm is 1:1 to 19:1.

In a further aspect, the present invention relates to pharmaceutical or dietary supplement including cetyl myristate or cetyl palmitate or combination of cetyl myristate and cetyl palmitate having compressibility index <20.

According to preceding claims, a) particles of cetyl myristate or cetyl palmitate or combination of cetyl myristate and cetyl palmitate or, b) powder mixtures of cetyl myristate or cetyl palmitate or combination of cetyl myristate and cetyl palmitate with an excipient or mixtures of excipients or, c) granules of cetyl myristate or cetyl palmitate or combination of cetyl myristate and cetyl palmitate are characterized by that compressibility index of particles is not greater than 20.

According to obtained results of batches, final blend of cetyl myristate or cetyl palmitate or combination of cetyl myristate and cetyl palmitate have an average of compressibility index of 14.82.

In the present invention, pharmaceutical or dietary supplement composition comprising cetyl myristate or cetyl palmitate or combination of cetyl myristate and cetyl palmitate can contain one or more excipients, such as diluents, binders, disintegrants, lubricants.

The amount of each type of additive employed, e.g. glidant, binder, disintegrant, filler or diluent and lubricant may vary within ranges conventional in the art.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, powdered cellulose, talc, tribasic calcium phosphate, mixtures thereof and the like.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

Flavourings are, but not limited to, cinnamon oil, essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehydeacacia, anise oil, benzaldehyde, caraway oil, cardamom (oil, tincture, spirit),cherry syrup, citric acid syrup, citric acid, clove oil, cocoa, cocoa syrup, coriander oil, dextrose, ethyl acetate, ethyl vanillin, fennel oil, ginger, glucose, glycerin, glycerrhiza, honey, lavender oil, lemon oil, mannitol, nutmeg oil, methyl salicylate, orange oil, orange flower water, peppermint (oil, spirit, water), raspberry, rose (oil, water), rosemary oil, saccharin sodium, Sarsaparilla syrup, spearmint oil, thyme oil, tolu balsam, vanilla ,vanilla tincture, tolu balsam syrup, wild cherry syrup and mixtures thereof and the like.

Viscosity increasing agents /suspending agents are, but not limited to, cellulose derivatives such as hydroxypropylcellulose, hydroxypropyl metylcellulose, methylcellulose, hydroxyethylcellulose, carboxymethylcellulose, sodium carboxymethylcellulose, microcrystalline cellulose, acacia, alginates, tragacanth, xanthan gum, bentonite, carbomer, carageenan, powdered cellulose, gelatin and mixtures thereof and the like. Xanthan gum is preferred.

Buffering components are added to stabilize the suspension to a desired pH range. Buffering agents are but not limited to, salts of week acids such as carbonates, citrates, gluconates, phosphate and tartrates and mixtures thereof and the like. Citric acid and sodium citrate are mostly used to stabilize pH.

Selection of excipients and amounts to use can be determined by the scientist. One or more these additives can be chosen and used by the artisan having regard to the particular desired properties of the solid dosage form.

Suitable pharmaceutical compositions include, but are not limited to, capsules, tablets, granules, powders and unit dose pockets.

The following examples are given for the purpose of illustration of the present invention and should not limit the scope of the invention.

### Example : Capsule Unit Formula and Manufacturing Process:

There is provided a process for the capsule preparation of a pharmaceutical form of cetyl myristate or cetyl palmitate or combination of cetyl myristate and cetyl palmitate, suitable for oral administration, said process comprising following steps:

a) 350.00 mg per capsule of active ingredients and 63.6 mg per capsule of diluent, 7.05 mg per capsule of disintegrant, 23.50 mg per capsule of binder are weighed and sifted using a suitable screen in Vibratory sifter.

b) Sifted diluents, active ingredients, disintegrant and binder are transferred to Granulator and premixed.

c) Granulation is performed with a granulation agent and granules are dried in Glatt Powder Coater and Granulator (GPCG).

d) Mill the granules retained on Vibratory sifter through suitable screen in a Quadro co-mill to break small lumps / larger granules.

e) Granules are stored in air tight HDPE container lined with double poly bag.

f) Fill and collect capsules after checking the following: Individual weight variation, group weight and disintegration time.

## Claims

1. A pharmaceutical or dietary supplement formulations of cetyl myristate or cetyl palmitate or combination of cetyl myristate and cetyl palmitate **characterized in that** said formulations are prepared by using of: a) particles of cetyl myristate or cetyl palmitate or combination of cetyl myristate and cetyl palmitate having particle size distribution in the range of 150 µm ≤PSD≤ 1600 µm or, b) powder mixtures of cetyl myristate or cetyl palmitate or combination of cetyl myristate and cetyl palmitate with an excipient or mixtures of excipients having particle size distribution in the range of 150 µm ≤PSD≤ 1600 µm or, c) granules of cetyl myristate or cetyl palmitate or combination of cetyl myristate and cetyl palmitate having particle size distribution in the range of 150 µm ≤PSD≤ 1600 µm wherein particle size distribution of granules, particles and powder mixtures is determined according to US Pharmacopeia General Chapter <786>.

2. Particles or granules or powder mixtures, as claimed in claim 1, have following particle size distribution: a-) 95% of particles are larger than 150 µm and, b-) 25% of particles are between 150 µm and 250 µm and, c-) 60% of particles are between 250 µm and 425µm and, d-) 60% of particles are larger than 425 µm.

3. According to preceding claims, a) particles of cetyl myristate or cetyl palmitate or combination of cetyl myristate and cetyl palmitate or, b) powder mixtures of cetyl myristate or cetyl palmitate or combination of cetyl myristate and cetyl palmitate with an excipient or mixtures of excipients or, c) granules of cetyl myristate or cetyl palmitate or combination of cetyl myristate and cetyl palmitate **characterized by** that compressibility index of particles is not greater than 20, wherein CI is determined according to US Pharmacopeia General Chapter <1174>.

4. According to claim 1 to 2,a) particles of cetyl myristate or cetyl palmitate or combination of cetyl myristate and cetyl palmitate or, b) powder mixtures of cetyl myristate or cetyl palmitate or combination of cetyl myristate and cetyl palmitate with an excipient or mixtures of excipients or, c) granules of cetyl myristate or cetyl palmitate or combination of cetyl myristate and cetyl palmitate **characterized in that** a-) ratio of quantity of ultrafine particles to quantity of coarse particles is 2:1 to 1:6, b-) ratio of quantity of coarse particles to quantity of fine particles is 5:1 to 5:12, c-) ratio of quantity of coarse particles to quantity of cumulative total particles is 1:19 to 3:2, d-) ratio of quantity of particles with particles larger than 250 µm to quantity of particles smaller than 250 µm is 1:1 to 19:1.

## Patentansprüche

1. Formulierungen von Arzneimittel oder Nahrungsergänzungsmittel von Cetylmyristat oder Cetylpalmitat oder Kombination von Cetylmyristat und Cetylpalmitat, **dadurch gekennzeichnet, dass** diese Formulierungen hergestellt werden unter Verwendung von: a) Partikeln von Cetylmyristat oder Cetylpalmitat oder Kombination von Cetylmyristat und Cetylpalmitat mit einer Partikelgrößenverteilung (PGV) im Bereich von 150 µm ≤PGV≤ 1600 µm oder, b) Pulvermischungen von Cetylmyristat und Cetylpalmitat oder Kombination von Cetylmyristat und Cetylpalmitat mit einem Hilfsstoff oder Mischungen von Hilfsstoffen, die eine Partikelgrößenverteilung im Bereich von 150 µm ≤PGV≤ 1600 µm aufweisen oder, c) Granulate von Cetylmyristat oder Cetylpalmitat oder Kombination von Cetylmyristat und Cetylpalmitat, die eine Partikelgrößenverteilung im Bereich von 150 µm ≤PGV≤ 1600 µm aufweisen, wobei die Partikelgrößenverteilung von Granulaten, Partikeln und Pulvermischungen nach General Chapter <786> der US-Pharmakopöe bestimmt wird.

2. Partikeln oder Granulate oder Pulvermischungen nach Anspruch 1, welche die folgende Partikelgrößenverteilung aufweisen: a-) 95% der Partikeln größer als 150 µm und, b-) 25% der Partikeln zwischen 150 µm und 250 µm und, c-) 60% der Partikeln zwischen 250 µm und 425 µm und, d-) 60% der Partikeln größer als 425 µm.

3. Nach nach einem der vorhergehenden Ansprüche, a) Partikeln von Cetylmyristat oder Cetylpalmitat oder Kombination von Cetylmyristat und Cetylpalmitat oder, b) Pulvermischungen von Cetylmyristat oder Cetylpalmitat oder Kombination von Cetylmyristat und Cetylpalmitat mit einem Hilfsstoff oder Mischungen von Hilfsstoffen oder, c) Granulate von Cetylmyristat oder Cetylpalmitat oder Kombination von Cetylmyristat und Cetylpalmitat, **dadurch gekennzeichnet, dass** der Kompressibilitätsindex (KI) der Partikeln nicht größer als 20 ist, wobei KI nach General Chapter <1174> der US-Pharmakopöe bestimmt wird.

4. Nach Ansprüchen 1 bis 2, a) Partikeln von Cetylmyristat oder Cetylpalmitat oder Kombination von Cetylmyristat und Cetylpalmitat oder, b) Pulvermischungen aus Cetylmyristat oder Cetylpalmitat oder Kombination von Cetylmyristat und Cetylpalmitat mit einem Hilfsstoff oder Mischungen von Hilfsstoffen oder, c) Granulate von Cetylmyristat oder Cetylpalmitat oder Kombination von Cetylmyristat und Cetylpalmitat, **dadurch gekennzeichnet, dass** a-) das Verhältnis der Menge von ultrafeinen Partikeln zur Menge von groben Partikeln 2:1 bis 1:6 beträgt, b-) das Verhältnis der Menge von groben Partikeln zur Menge von feinen Partikeln 5:1 bis 5:12 beträgt, c-) das Verhältnis der Menge von groben Partikeln zur kumulativen Gesamtmenge an Partikeln 1:19 bis 3:2 beträgt, d-) das Verhältnis der Menge von Partikeln größer als 250 µm zur Menge von Partikeln kleiner als 250 µm 1:1 bis 19:1 beträgt.

## Revendications

1. Une formule pharmaceutique ou de supplément alimentaire composée de myristate de cétyle ou palmitate de cétyle, ou une combinaison de myristate de cétyle et palmitate de cétyle, **caractérisée par** une préparation avec : a) particules de myristate de cétyle ou palmitate de cétyle, ou une combinaison de de myristate de cétyle et palmitate de cétyle, de tailles de particules distribuées entre 150 µm ≤ distribution granulométrique ≤ 1600 µm ou, b) mélanges de poudres de de myristate de cétyle ou palmitate de cétyle, ou une combinaison de myristate de cétyle et palmitate de cétyle, avec un excipient ou un mélange d'excipients, de tailles de particules distribuées entre 150 µm ≤distribution granulométrique≤ 1600 µm ou, c) granulés de de myristate de cétyle ou palmitate de cétyle, ou une combinaison de myristate de cétyle et palmitate de cétyle, de tailles de particules distribuées entre 150 µm ≤ distribution granulométrique ≤ 1600 µm, où la distribution des tailles de particules des granulés, particules et mélanges de poudres est déterminée selon la Pharmacopée Américaine, Chapitre <786>.

2. Particules ou granulés ou mélanges de poudres, selon la revendication 1, de distribution de tailles de particules suivante : a-) 95% des particules sont supérieures à 150 µm et, b-) 25% des particules se trouvent entre 150 µm et 250 µm et, c-) 60% des particules se trouvent entre 250 µm et 425µm et, d-) 60% des particules sont supérieures à 425 µm.

3. Selon les revendications précédentes, a) particules de myristate de cétyle ou palmitate de cétyle, ou une combinaison de myristate de cétyle et palmitate de cétyle ou, b) mélanges de poudres de myristate de cétyle ou palmitate de cétyle, ou une combinaison de myristate de cétyle et palmitate de cétyle, avec un excipient ou un mélange d'excipients ou, c) granulés de myristate de cétyle ou palmitate de cétyle, ou une combinaison de myristate de cétyle et palmitate de cétyle, **caractérisés par** un index de compressibilité des particules non supérieur à 20 où l'IC est déterminé selon la Pharmacopée américaine, Chapitre <1174>.

4. Selon les revendications 1 à 2, a) particules de myristate de cétyle ou palmitate de cétyle, ou une combinaison de myristate de cétyle et palmitate de cétyle, b) mélanges de poudres de myristate de cétyle ou palmitate de cétyle, ou une combinaison de myristate de cétyle et palmitate de cétyle, avec un excipient ou un mélange d'excipients ou, c) granulés de myristate de cétyle ou palmitate de cétyle, ou une combinaison de myristate de cétyle et palmitate de cétyle, **caractérisés par** une a-) proportion entre particules ultrafines et particules grossières de 2:1 à 1:6, b-) proportion entre particules grossières et particules fines de 5:1 à 5:12, c-) proportion entre particules grossières et total des particules de 1:19 à 3:2, d-) proportion entre les particules supérieures à 250 µm et les particules inférieures à 250 µm de 1:1 à 19:1.
